# EUROPEAN PATENT SPECIFICATION

(11) **EP 3 908 345 B1**
(45) Date of publication and mention of the grant of the patent: **21.10.2026**
(21) Application number: 20702378.9
(22) Date of filing: 13.01.2020
(51) Int. Cl.: A61M 1/34

(54) **PLASMA DETOXIFICATION SYSTEMS**
SYSTEME ZUR PLASMAENTGIFTUNG
SYSTÈMES DE DÉCONTAMINATION DU PLASMA

(30) Priority: 11.01.2019 US 201962791617 P
(43) Date of publication of application: 17.11.2021
(60) Divisional application: 26205366.3
(73) Proprietor: Marker Holdings AG, 6300 Zug (CH)
(72) Inventor: WENTHOLD, Randy, Menahga, Minnesota 56464 (US)
(74) Representative: Haseltine Lake Kempner LLP
(86) International application number: PCT/IB2020/050236
(87) International publication number: WO 2020/144664

(56) References cited:
- US-A- 4 388 163
- US-A1- 2001 051 106
- US-A1- 2004 228 829
- US-A1- 2005 281 809
- US-A1- 2012 267 290
- US-B2- 8 038 638

## Description

### CROSS-REFERENCE TO RELATED APPLICATIONS

This application claims priority benefit of U.S. Provisional Patent Application Serial No. 62/791,617, filed January 11, 2019.

### TECHNICAL FIELD

The present disclosure provides, *inter alia,* methods, systems, and devices for plasma detoxification using a closed fluid circuit.

### BACKGROUND

Sepsis is the primary cause of death in the intensive care unit and more than 35% of patients are admitted with sepsis or develop it during their intensive care unit stay. Hospital mortality rates are 27%, reaching 54% in the case of septic shock. Extracorporeal blood purification therapies have been proposed to improve outcomes for patients with sepsis. These therapies are based on the principle that removal of inflammatory mediators or bacterial toxins (or both) from the blood will favorably modulate the host inflammatory response. Recently, significant technological progress has greatly broadened the spectrum of techniques available for blood purification. Promising results have been reported with high-volume hemofiltration (HVHF), cascade hemofiltration, hemoadsorption, plasmapheresis, coupled plasma filtration adsorption (CPFA), high-adsorption hemofiltration, and high-cutoff (HCO) hemodialysis/hemofiltration. However, these techniques have not entered into mainstream clinical practice around the world.

Many doctors view sepsis as a three-stage syndrome, starting with sepsis and progressing through severe sepsis to septic shock. The goal is to treat sepsis during its early stage, before it becomes more dangerous.

Plasma detoxification systems with extracorporeal circuits having plasma filter devices incorporated therein are known in the art. See, e.g., U.S. Patent No. 8,038,638 (Hemolife Medical) (hereinafter "the '638 patent") and European Patent No. EP 0787500 A1 (Bellco) (hereinafter "the '500 patent"). These plasma detoxification systems are described as effective to treat sepsis, renal failure, and liver failure. There are several deficiencies with these systems. For example, an acute renal failure pump that must possess a plasma adsorption mode is required in combination with the plasma separation filter device and the adsorptive toxin removal device. These systems also require complex tubing connections to be effective. Furthermore, these systems must be used with anticoagulation to function. Sodium heparin, which is the anticoagulant used in these systems, is expensive and difficult to dose during the therapy, and can be an issue for patients subject to bleeding.

No one extracorporeal system known in the art has been successful in the market place due to the esoteric tubing requirements and sodium heparin anticoagulation requirements used to manage the treatment. Previous systems also have made sepsis treatment more difficult to manage by attempting to combine fluid removal via a hemofilter device. Anticoagulation control of the patient's blood without excess plasma fluid removal is itself difficult. Thus, adding excess plasma fluid removal while simultaneously controlling the patient's blood clotting via sodium heparin anticoagulation is a difficult clinical practice and a reason why the current systems known in the art have not been successful in the market. Intensive Care Unit (ICU) treatment associated with all existing extracorporeal devices introduced for treatment is another reason why previous systems have not been successful in the market. Therefore, there remains a need for a safe and effective extracorporeal system for plasma detoxification.

US 2001 051106 A1 discloses a hemofiltration system for a mammal comprising a hemofilter and an adsorbent device. The hemofilter removes ultrafiltrate from a blood stream extracted from the mammal to create a filtered blood stream and an ultrafiltrate stream. The adsorbent device is comprised of one or more adsorbent materials and is used to adsorb inflammatory mediators from the ultrafiltrate stream received from the hemofilter.

The present invention is directed to overcoming these and other deficiencies in the art.

### SUMMARY OF THE CLAIMED INVENTION

The present invention claims a system according to claim 1. Preferred embodiments are claimed in the dependent claims 2-8.

### SUMMARY OF THE DISCLOSURE

The present disclosure provides, *inter alia,* methods, systems, and devices for plasma detoxification using a closed fluid circuit. As described herein, the methods, systems, and devices of the present disclosure provide an extracorporeal system that can be used to safely remove toxins from plasma in patients suffering from many forms of sepsis, liver failure, acute respiratory distress, viral infections, poisoning, inflammation, and many other diseases and conditions treatable by plasma detoxification. In accordance with the present disclosure, the methods, systems, and devices provided herein are improvements over the existing art because they can use standard venous blood access with a centrifugal apheresis pump or similar device, thereby enabling therapeutic treatments to be administered as an out-patient type service instead of being limited to an ICU treatment.

In one aspect, the present disclosure provides a system for removing cytokines and other substances from blood of a subject in a closed fluid circuit. This system includes components effective to perform the following method steps: (i) passing venous blood from the subject through a plasma separator, thereby separating the blood into blood cells and plasma; (ii) passing the plasma received from the plasma separator through an adsorption chamber located in the circuit to form processed plasma, wherein materials in the adsorption chamber adsorb cytokines in the plasma to form the processed plasma, said materials comprising, by weight, 50-70% activated carbon and 30-50% non-ionic resin; (iii) combining the processed plasma, received directly from the adsorption chamber, with the blood cells in a combining chamber to form processed blood, without exchanging any of the plasma for another fluid; and (iv) transfusing the processed blood from the circuit directly into the subject, wherein no fluid besides the subject's blood is added to the circuit before the transfusing of the processed blood into the subject is completed.

In another aspect, the present disclosure provides a system for use in the therapeutic treatment of a disease or condition selected from the group consisting of sepsis, liver failure, viral infection, acute respiratory distress, renal failure, inflammation, poisoning, drug overdose, autoimmune disease, tick-borne illness, chemical or nerve agent exposure, burn biliary obstruction, post-surgery inflammation, bacterial infection, complications caused by smoke inhalation, complications as a result of any form of injury or trauma, and complications as a result of any form of cancer or cancer treatment, wherein said system comprises a plasma separator, an adsorption chamber, and a combining chamber, and wherein said system is used for said therapeutic treatment of the disease or condition by removing cytokines and other substances from blood of a subject in a closed fluid circuit, said system being effective to perform the following method steps: (i) passing venous blood from the subject through the plasma separator, thereby separating the blood into blood cells and plasma; (ii) passing the plasma received from the plasma separator through the adsorption chamber located in the circuit to form processed plasma, wherein materials in the adsorption chamber adsorb cytokines in the plasma to form the processed plasma, said materials comprising, by weight, 50-70% activated carbon and 30-50% non-ionic resin; (iii) combining the processed plasma, received directly from the adsorption chamber, with the blood cells in the combining chamber to form processed blood, without exchanging any of the plasma for another fluid; and (iv) transfusing the processed blood from the circuit directly into the subject, wherein no fluid besides the subject's blood is added to the circuit before the transfusing of the processed blood into the subject is completed.

In another aspect, the present disclosure provides for the use of an adsorption chamber for the manufacture of a system according to the present disclosure for the therapeutic treatment of a disease or condition selected from the group consisting of sepsis, liver failure, viral infection, acute respiratory distress, renal failure, inflammation, poisoning, drug overdose, autoimmune disease, tick-borne illness, chemical or nerve agent exposure, burn biliary obstruction, post-surgery inflammation, bacterial infection, complications caused by smoke inhalation, complications as a result of any form of injury or trauma, and complications as a result of any form of cancer or cancer treatment.

In another aspect, the present disclosure provides a method of removing cytokines and other substances from blood of a subject in a closed fluid circuit. This method involves the steps of: (i) passing venous blood from the subject through a plasma separator, thereby separating the blood into blood cells and plasma; (ii) passing the plasma received from the plasma separator through an adsorption chamber located in the circuit to form processed plasma, wherein materials in the adsorption chamber adsorb cytokines in the plasma to form the processed plasma, said materials comprising, by weight, 50-70% activated carbon and 30-50% non-ionic resin; (iii) combining the processed plasma, received directly from the adsorption chamber, with the blood cells in a combining chamber to form processed blood, without exchanging any of the plasma for another fluid; and (iv) transfusing the processed blood from the circuit directly into the subject, wherein no fluid besides the subject's blood is added to the circuit before the transfusing of the processed blood into the subject is completed.

In another aspect, the present disclosure provides a method for therapeutic treatment of a subject. This method involves performing the method of removing cytokines and other substances from blood of a subject in a closed fluid circuit as described herein, thereby providing therapeutic treatment to the subject.

In certain embodiments, the methods, systems, and devices of the present disclosure involves the use of an adsorptive toxin removal device with a centrifugal apheresis pump to effectively detoxify plasma from patients suffering from various diseases. As described in more detail herein, the methods, systems, and devices of the present disclosure are advantageous over the existing art in that they reduce the number and complexity of devices, tubing, and components required for treatment. Furthermore, the methods, systems, and devices of the present disclosure can use anticoagulant citrate dextrose solution ACD-A, rather than being limited to using sodium heparin for the anticoagulant. The methods, systems, and devices of the present disclosure are also advantageous in that they incorporate effective device design for easy manufacture, and enable an assembly method to manufacture small scale devices for valuable scale-up laboratory studies or for use with small to medium sized patient's, including children.

### BRIEF DESCRIPTION OF THE DRAWINGS

The accompanying drawings illustrate a number of exemplary embodiments and are a part of the specification. Together with the following description, these drawings demonstrate and explain various principles of the instant disclosure.
**FIG. 1** is a schematic of an exemplary embodiment of a system for removing cytokines and other substances from blood of a subject in a closed fluid circuit, as provided in the present disclosure.
**FIG. 2** is a schematic of an exemplary embodiment of an adsorption chamber for use in the methods, systems, and devices for removing cytokines and other substances from blood of a subject in a closed fluid circuit, as provided in the present disclosure.

Throughout the drawings, identical reference characters and descriptions indicate similar, but not necessarily identical, elements. While the exemplary embodiments described herein are susceptible to various modifications and alternative forms, specific embodiments have been shown by way of example in the drawings and will be described in detail herein. However, the exemplary embodiments described herein are not intended to be limited to the particular forms disclosed. Rather, the instant disclosure covers all modifications, equivalents, and alternatives falling within the scope of the appended claims.

### DETAILED DESCRIPTION OF EXEMPLARY EMBODIMENTS

The instant disclosure is directed to, *inter alia,* methods, systems, and devices for plasma detoxification using a closed fluid circuit. As described herein, the methods, systems, and devices of the present disclosure provide an extracorporeal system that can be used to safely remove cytokines and other toxins and unwanted substances from the plasma of patients suffering from various diseases and conditions including, as described in more detail herein.

As used herein, the term "cytokines" refers to a broad category of small proteins (~5-20 kDa) that are important in cell signaling. Cytokines may include, without limitation, chemokines, interferons, interleukins, lymphokines, and tumor necrosis factors. Cytokines can be produced by a broad range of cells, including, for example, immune cells like macrophages, B lymphocytes, T lymphocytes and mast cells, as well as endothelial cells, fibroblasts, and various stromal cells.

As used herein, the terms "toxins" and "substances" (also referred to as "unwanted substances") refer to any organic or inorganic compound that, when present in a subject's blood above a tolerable threshold, causes an adverse effect on the subject Representative examples of toxins in accordance with the present disclosure include, without limitation, cytokines including interleukins (including but not limited to, IL-3), interferons, tumor necrosis factors alpha or gamma, soluble proteins, bilirubin, creatinine, amino acids, nucleic acids, bacterial toxins including endotoxins, exotoxins, lipopolysacccharides, cellular enzymes, bacterial cell wall components and pharmaceuticals such as acetaminophen.

In accordance with the present disclosure, the methods, systems, and devices provided herein enable standard venous blood access with a centrifugal apheresis pump or similar device, thereby enabling therapeutic treatments to be administered in an out-patient type manner rather than such therapeutic treatments being limited to an ICU setting.

In one aspect, the present disclosure is directed to a system for removing cytokines and other substances from blood of a subject in a closed fluid circuit. As described in more detail herein, the system is an extracorporeal plasma detoxification system that includes components and devices that are effective to carry out the methods of the present disclosure. At a minimum the system of the present disclosure includes the following components and/or devices: a plasma separator; an adsorption chamber; and a combining chamber, each of which is described in more detail herein. The system of the present disclosure is effective to remove cytokines and other substances from the blood of a subject by assisting in carrying out the following method steps of the present disclosure: (i) passing venous blood from the subject through a plasma separator, thereby separating the blood into blood cells and plasma; (ii) passing the plasma received from the plasma separator through an adsorption chamber located in the circuit to form processed plasma, wherein materials in the adsorption chamber adsorb cytokines in the plasma to form the processed plasma, said materials comprising, by weight, 50-70% activated carbon and 30-50% non-ionic resin; (iii) combining the processed plasma, received directly from the adsorption chamber, with the blood cells in a combining chamber to form processed blood, without exchanging any of the plasma for another fluid; and (iv) transfusing the processed blood from the circuit directly into the subject, wherein no fluid besides the subject's blood is added to the circuit before the transfusing of the processed blood into the subject is completed.

In another aspect, the present disclosure is directed to a system for use in the therapeutic treatment of a disease or condition selected from the group consisting of sepsis, liver failure, viral infection, acute respiratory distress, renal failure, inflammation, poisoning, drug overdose, autoimmune disease, tick-borne illness, chemical or nerve agent exposure, burn biliary obstruction, post-surgery inflammation, bacterial infection, complications caused by smoke inhalation, complications as a result of any form of injury or trauma, and complications as a result of any form of cancer or cancer treatment, wherein said system comprises a plasma separator, an adsorption chamber, and a combining chamber, and wherein said system is used for said therapeutic treatment of the disease or condition by removing cytokines and other substances from blood of a subject in a closed fluid circuit, said system being effective to perform the following method steps: (i) passing venous blood from the subject through the plasma separator, thereby separating the blood into blood cells and plasma; (ii) passing the plasma received from the plasma separator through the adsorption chamber located in the circuit to form processed plasma, wherein materials in the adsorption chamber adsorb cytokines in the plasma to form the processed plasma, said materials comprising, by weight, 50-70% activated carbon and 30-50% non-ionic resin; (iii) combining the processed plasma, received directly from the adsorption chamber, with the blood cells in the combining chamber to form processed blood, without exchanging any of the plasma for another fluid; and (iv) transfusing the processed blood from the circuit directly into the subject, wherein no fluid besides the subject's blood is added to the circuit before the transfusing of the processed blood into the subject is completed.

In another aspect, the present disclosure is directed to the use of an adsorption chamber for the manufacture of a system according to the present disclosure for the therapeutic treatment of a disease or condition selected from the group consisting of sepsis, liver failure, viral infection, acute respiratory distress, renal failure, inflammation, poisoning, drug overdose, autoimmune disease, tick-borne illness, chemical or nerve agent exposure, burn biliary obstruction, post-surgery inflammation, bacterial infection, complications caused by smoke inhalation, complications as a result of any form of injury or trauma, and complications as a result of any form of cancer or cancer treatment.

In another aspect, the present disclosure is directed to a method of removing cytokines and other substances from blood of a subject in a closed fluid circuit. This method involves passing venous blood from the subject through a plasma separator. This step results in separating the blood into blood cells and plasma. The plasma received from the plasma separator is passed through an adsorption chamber located in the circuit to form processed plasma. The adsorption chamber is configured to include materials that adsorb cytokines in the plasma to form the processed plasma. More specifically, these adsorption materials contained in the adsorption chamber include, by weight, 50-70% activated carbon and 30-50% non-ionic resin. After passing through the adsorption chamber, the processed plasma is received directly from the adsorption chamber and combined with the subject's blood cells in a combining chamber to form processed blood. This is done without exchanging any of the plasma for another fluid. The method then involves transfusing the processed blood from the circuit directly into the subject. During the transfusing step, no fluid besides the subject's blood is added to the circuit before the transfusing of the processed blood into the subject is completed.

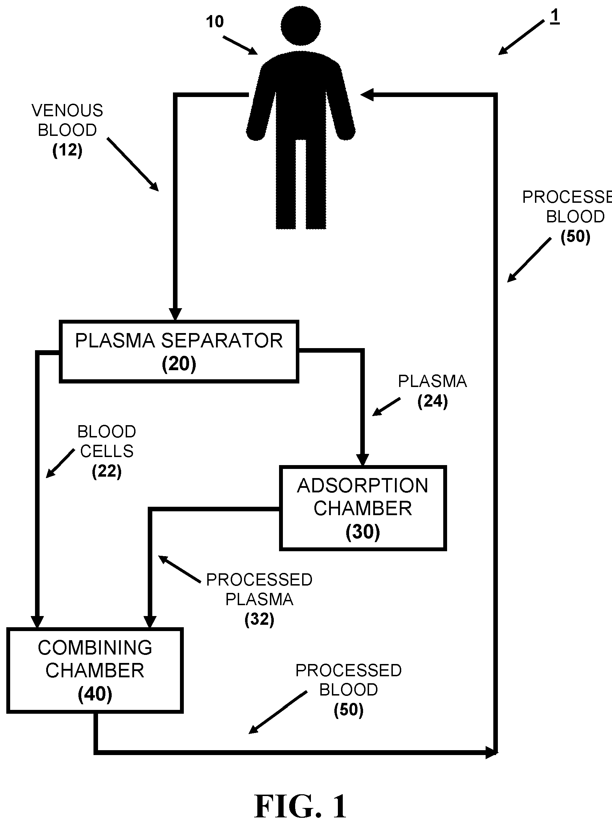

**FIG. 1** illustrates an exemplary embodiment of a system for removing cytokines and other substances from the blood of a subject in a closed fluid circuit, as provided in the present disclosure. As shown in **FIG. 1****,** system **1** includes plasma separator **20,** adsorption chamber **30,** and combining chamber **40.** During operation of system **1,** venous blood **12** is taken from subject **10** and passed through plasma separator **20,** thereby separating venous blood **12** into blood cells **22** and plasma **24.** Plasma **24** is received from plasma separator **20** and passed through adsorption chamber **30** to form processed plasma **32.** Adsorption chamber **30** includes materials that adsorb cytokines and optionally other substances in plasma **24** to form processed plasma **32.** As provided herein, the adsorption materials include, by weight, 50-70% activated carbon and 30-50% non-ionic resin. Processed plasma **32** is received directly from adsorption chamber **30** and combined with blood cells **22** in combining chamber **40** to form processed blood **50,** without exchanging any of plasma **24** or processed plasma **32** for another fluid. Processed blood **50** is then transfused from the closed fluid circuit of system **1** directly back into subject **10.** No fluid besides venous blood **12** of subject **10** is added to the closed fluid circuit of system **1** before transfusing of processed blood **50** into subject **10** is completed.

As used herein, a "closed fluid circuit" refers to an extracorporeal plasma detoxification system that is configured as a closed loop to receive venous blood from a subject and return the processed blood to the same subject, after processing the blood through a series of devices as described herein. These devices include a plasma separator, an adsorption chamber, and a combining chamber as described herein.

As used herein, a "plasma separator" refers to a device suitable for use in separating venous blood from a subject into blood cells and plasma. Suitable examples of plasma separators for use in the methods, systems, and devices of the present disclosure include, without limitation, the following: HAEMOSELECT^{®} M 0.3 plasma filter (B. Braun Medical Inc.), HAEMOSELECT^{®} L 0.5 plasma filter (B. Braun Medical Inc.), PLASMAFLUX^{®} P1 dry plasma filter (Fresenius Medical Care), PLASMAFLUX^{®} P2 dry plasma filter (Fresenius Medical Care), PLASMART^{™} 50 plasma filter (MEDICA S.p.A.), PLASMART^{™} 100 plasma filter (MEDICA S.p.A.), PLASMART^{™} 200 plasma filter (MEDICA S.p.A.), PLASMART^{™} 400 plasma filter (MEDICA S.p.A.), PLASMART^{™} 600 plasma filter (MEDICA S.p.A.), PLASMART^{™} 700 plasma filter (MEDICA S.p.A.), PLASMART^{™} 1000 plasma filter (MEDICA S.p.A.), PLASMAFLO^{™} OP-02W(L) hollow fiber plasma separator (Asahi Kasei Medical Co., Ltd.), PLASMAFLO^{™} OP-05W(L) hollow fiber plasma separator (Asahi Kasei Medical Co., Ltd.), PLASMAFLO^{™} OP-08W(L) hollow fiber plasma separator (Asahi Kasei Medical Co., Ltd.), PRISMAFLEX^{®} TPE 1000 set plasma filter system (Baxter/Gambro), and PRISMAFLEX^{®} TPE 2000 set plasma filter system (Baxter/Gambro).

As used herein, an "adsorption chamber" refers to a device suitable for use in removing cytokines and other substances from the blood of a subject. As described herein, the adsorption chamber of the present disclosure contains adsorption materials that adsorb cytokines in the plasma to form the processed plasma. As described in more detail herein, the adsorption chamber can also be configured to remove toxins other than cytokines from the blood of a subject.

More specifically, the adsorption chamber of the present disclosure contains adsorption materials that include, by weight, 50-70% activated carbon and 30-50% non-ionic resin, as described in more detail herein. Although the adsorption chamber must include the aforementioned activated carbon and non-ionic resin, it can also include other components, as long as they do not interfere with the functionality of the adsorption chamber as described herein.

As used herein, the term "adsorption materials" refers to the materials contained in the adsorption chamber that are effective to remove cytokines and other substances of interest from the blood of a subject. In certain instances, the term "materials" may be used to denote "adsorption materials" of the present disclosure. More specifically, the adsorption materials of the present disclosure include activated carbon and non-ionic resins. When used in the adsorption chamber of the present disclosure, the adsorption materials are present in an amount, by weight, of 50-70% activated carbon and 30-50% non-ionic resin. In one embodiment, the activated carbon includes at least one activated carbon material selected from, for example, uncoated coconut shell granule charcoal, uncoated organic granule charcoal, uncoated synthetic carbon, and the like. Suitable non-ionic resins can include, without limitation, at least one resin material selected from a non-ionic aliphatic ester resin, a non-ionic polystyrene divinyl benzene resin, an agarose media with hydrophobic interactive chromatography, and other non-biologic adsorptive resins. A suitable non-ionic aliphatic ester resin can include, without limitation, AMBERLITE^{®} XAD-7HP. A suitable non-ionic polystyrene divinyl benzene resin can include, without limitation, AMBERCHROM^{®} GC300C.

The non-ionic resins suitable for use in the methods, systems, and devices of the present disclosure are further described below.

In certain embodiments of the methods, systems, and devices of the present disclosure, non-ionic exchange resins are exclusively used in accordance with the teachings of the present disclosure because they will not bind (and thus removed from the blood) essential cations and anions such as, but not limited to, calcium, magnesium, sodium, potassium, chloride, carbonates, and other ionic species. This is important when recirculating patient's plasma through an adsorptive device since changes in electrolytes results in changes in osmolality of a patient's blood chemistry which is not desired.

Specific non-limiting examples of non-ionic exchange resins suitable for use with the methods, systems, and devices of the present disclosure can include, without limitation, AMBERLITE^{™} XAD-7 HP, AMBERCHROM^{™} CG300-C, and hydrophobic interaction chromatography resins (Butyl-S Sepharose 6, Butyl Sepharose 4, Capto Pheno, Capto Butyl, Capto Octyl, Capto Phenyl ImRes, Capto Butyl ImpRes, Phenyl Sepharose High Performance, Butyl Sepharose High Performance, Phenyl Sepharose 6 FastFlow low-sub, Phenyl Sepharose 6 FastFlow high-sub).

AMBERLITE^{™} is a group of polymeric synthetic resins made by the Rohm and Haas Company having a North American headquarters at 100 Independence Mall West Philadelphia, PA 19106-2399. AMBERLITE^{™} resins are available worldwide through a distributor network know to those skilled in the art. In one specific embodiment, the present disclosure involves the use of AMBERLITE^{™} XAD-7 HP, which is an aliphatic ester resin having an average surface area of approximately 500 m²/g and an average pore size of approximately 450 Angstroms and a mean diameter of approximately 560 microns.

AMBERCHROME^{™} CG300-G is a synthetic non-ionic exchange resin, also manufactured by Rohm and Haas, made from polystyrene divinyl benzene having an average surface area of approximately 700 m²/g with an average pore size of 300 Angstroms; mean particle diameter ranges from approximately 35 microns to approximately 120 microns.

As used herein, hydrophobic interaction chromatography resins have particle diameters between 30 and 200 microns and are media produced by GE Healthcare Bio Sciences AB, Bjorkgaten 30, 751 84 Uppsala Sweden.

In one embodiment, the adsorption chamber is constructed from a polymer including, without limitation, polycarbonate, polypropylene, a Lexan co-polymer, polytetrafluoroethylene, and other medical grade polymers suitable for injection or blow molding.

In another embodiment, the adsorption chamber and/or the materials contained in the adsorption chamber are coated with human serum albumin and an anticoagulant added to physiological saline as a solution prior to clinical use.

Suitable anticoagulants for use in the methods, systems, and devices of the present disclosure include, without limitation, sodium heparin and citrate dextrose solution ACD-A.

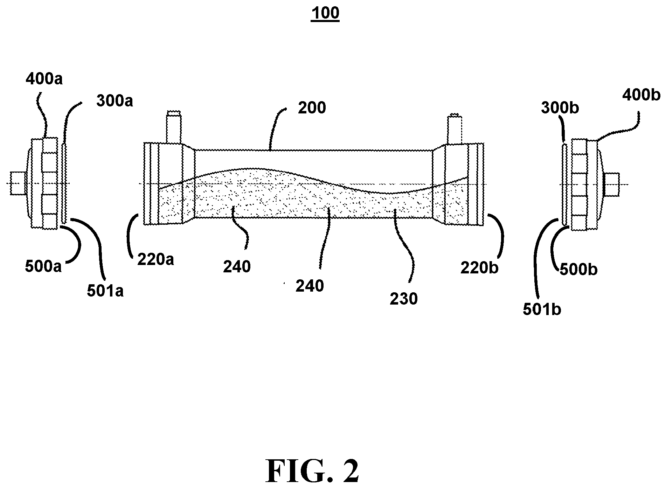

**FIG. 2** illustrates an exemplary embodiment of an adsorption chamber for use in the methods, systems, and devices of the present disclosure. As shown in **FIG. 2****,** adsorption chamber **100** includes housing **200,** porous membranes **300a, 300b,** and endcaps **400a, 400b.** Housing **200** includes hollow tube **210** with opposing open ends **220a, 220b.** Housing **200** contains activated carbon **230** and one or more non-ionic resin **240.** Porous membrane filters **300a, 300b** covers each of ends **220a, 220b** of housing **200,** each porous membrane filter **300a, 300b** creating a barrier for maintaining activated carbon **230** and non-ionic resins **240** within housing **200** while allowing for passage therethrough of plasma during performance of the method of the present disclosure. Endcaps **400a, 400b** fitted to each of ends **220a, 220b** of housing **200,** where each endcap **400a, 400b** is configured to keep its corresponding porous membrane filter **300a, 300b** in place and to maintain a seal between each endcap **400a, 400b** and the corresponding end **220a, 220b** of housing **200.**

In certain embodiments of the adsorption chamber of the present disclosure, each endcap includes a groove molded into its entire inner circumference. As provided herein, the groove is configured to facilitate mating of each endcap with the corresponding end of the housing.

In certain embodiments, the groove is configured to receive a quantity of adhesive. As shown in the exemplary embodiment adsorption chamber **100** of **FIG. 2****,** adhesive **500a, 500b** is deposited in the groove so as to aid in adhering porous membrane filters **300a, 300b** to endcap **400a, 400b.** In certain embodiments, another quantity of adhesive **501a, 501b** is deposited between each endcap **400a, 400b** and its corresponding porous membrane filter **300a, 300b** to provide further adhesion between endcap **400a, 400b** and the corresponding end **220a, 220b** of the housing **200.**

In certain embodiments, the ends of the housing are threaded and the corresponding endcaps are also threaded so as to mate with one another.

In certain embodiments, the housing is in the form of a tube comprising at least one of polypropylene, polytetrafluoroethylene, or other medical grade tubing materials.

In another aspect, the present disclosure is directed to a method for therapeutic treatment of a subject involving the use of the methods, systems, and devices of the present disclosure for removing cytokines and other substances from the blood of the subject, thereby providing therapeutic treatment to the subject. In one embodiment, the therapeutic treatment of the present disclosure can be administered via use of a standard venous access in an outpatient treatment setting.

In accordance with the method for therapeutic treatment of the present disclosure, the therapeutic treatment can be for a disease or condition that can include, without limitation, sepsis, liver failure, viral infection, acute respiratory distress, renal failure, inflammation, poisoning, drug overdose, autoimmune disease, tick-borne illness, chemical or nerve agent exposure, burn biliary obstruction, post-surgery inflammation, bacterial infection, complications caused by smoke inhalation, complications as a result of any form of injury or trauma, and complications as a result of any form of cancer or cancer treatment.

As provided herein, the autoimmune disease can include, without limitation, inflammatory arthritis, psoriasis, Crohn's disease, ulcerative colitis, inflammatory bowel disease, uveitis, and the like.

As provided herein, the inflammation can be treated, without limitation, using an age defying anti-inflammation application such as cosmetic, pain, and discomfort applications.

In a specific embodiment, the present disclosure provides an extracorporeal plasma detoxification system that can remove toxins associated with and resulting from sepsis, liver failure, renal failure, acute respiratory distress, auto immune, viral, poison, tick, pancreatic cancer bilirubin management, post-surgery inflammation management and other inflammation disease from the plasma of patients in need of therapeutic treatment. One embodiment of a system of the present disclosure can include, without limitation, an extracorporeal system that can generally be used to remove blood via a catheter, AV fistula or graft from a patient in need of plasma detoxification. Blood is removed from a large vein of a patient via one lumen of a conventional dual lumen catheter connected to a centrifugal apheresis pump where the blood cells are separated from the plasma fraction of the blood. The separated blood leaves the centrifugal apheresis pump and can continue in one of two pathways. Blood cells are returned to the patient while the separated plasma enters and passes through the adsorption column which is the toxin removal device of the present disclosure, which contains a mixture of adsorbent materials. The adsorptive toxin removal device removes both protein-bound and soluble toxins. After leaving the adsorbent column, the plasma flow is recombined with the patient's blood cells.

The headings provided herein are not limitations of the various aspects or embodiments of the invention, which can be had by reference to the specification as a whole. Accordingly, the terms defined immediately below are more fully defined by reference to the specification as a whole.

The terms "a" and "an" and "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided herein is intended merely to better illuminate the invention and does not pose a limitation on the scope of the invention otherwise claimed.

Preferred embodiments of this invention are described herein, including the best mode known to the inventors for carrying out the invention. Of course, variations on those preferred embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications of the subject matter recited in the claims appended hereto as permitted by applicable law. Moreover, any combination of the above-described elements in all possible variations thereof is encompassed by the invention unless otherwise indicated herein or otherwise clearly contradicted by context.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein.

Other advantages which are obvious and which are inherent to the disclosure will be evident to one skilled in the art. It will be understood that certain features and subcombinations are of utility and may be employed without reference to other features and subcombinations. This is contemplated by and is within the scope of the claims. Since many possible embodiments may be made of the disclosure without departing from the scope thereof, it is to be understood that all matter herein set forth or shown in the accompanying drawings is to be interpreted as illustrative and not in a limiting sense.

## Claims

1. A system for removing cytokines and other substances from blood of a subject (10) in a closed fluid circuit, said system comprising a plasma separator (20), an adsorption chamber (30) located in the circuit and a combining chamber (40), wherein the adsorption chamber comprises materials comprising, by weight, 50-70% activated carbon (230) and 30-50% non-ionic resin (240), and wherein the system is effective and configured to perform the following method steps:
(i) pass venous blood (12) from the subject through the plasma separator, wherein the plasma separator is configured to separate the blood into blood cells (22) and plasma (24);
(ii) pass the plasma directly received from the plasma separator through the adsorption chamber located in the circuit to form processed plasma (32), wherein materials in the adsorption chamber are configured to adsorb cytokines in the plasma to form the processed plasma; and
(iii) combine the processed plasma, received directly from the adsorption chamber, with the blood cells in the combining chamber, wherein the combining chamber is configured to form processed blood (50), without exchanging any of the plasma for another fluid; and configured such that the processed blood from the circuit can be transfused directly into the subject,
wherein no fluid besides the subject's blood is added to the circuit before transfusion of the processed blood into the subject is to be completed; and
wherein the plasma separator is a centrifugal apheresis pump.

2. The system according to claim 1, wherein the non-ionic resin comprises at least one resin material selected from the group consisting of a non-ionic aliphatic ester resin, a non-ionic polystyrene divinyl benzene resin, an agarose media with hydrophobic interactive chromatography, and other non-biologic adsorptive resins.

3. The system according to claim 2, wherein
(i) the non-ionic aliphatic ester resin is AMBERLITE^{®} XAD-7HP., or
(ii) the non-ionic polystyrene divinyl benzene resin is AMBERCHROM^{®} GC300C.or
(iii) the activated carbon comprises at least one activated carbon material selected from the group consisting of uncoated coconut shell granule charcoal, uncoated organic granule charcoal, and uncoated synthetic carbon

4. The system according to claim 1, wherein the adsorption chamber is constructed from a polymer selected from the group consisting of polycarbonate, polypropylene, a Lexan co polymer, polytetrafluoroethylene, and other medical grade polymers suitable for injection or blow molding.

5. The system according to claim 1, wherein the adsorption chamber comprises: a housing (200) comprising a hollow tube with opposing open ends, said housing containing the activated carbon and non-ionic resin;
porous membrane filters (300a,300b) covering each of the ends (220a,220b) of the housing, each porous membrane filter creating a barrier for maintaining the activated carbon and non-ionic resin within the housing while allowing for passage therethrough of the plasma; and
endcaps (400a,400b) fitted to each of the ends of the housing, wherein each endcap is configured to keep its corresponding porous membrane filter in place and to maintain a seal between the endcap and the corresponding end of the housing.

6. The system according to claim 5, wherein
(i) each endcap includes a groove molded into its entire inner circumference, said groove being configured to facilitate mating of each endcap with the corresponding end of the housing, optionally
wherein said groove is configured to receive a quantity of adhesive, and said adhesive being deposited in the groove so as to aid in adhering the porous membrane filter to the endcap, and further optionally
wherein another quantity of adhesive is deposited between each endcap and its corresponding porous membrane filter to provide further adhesion between the endcap and the corresponding end of the housing, or
(ii) the ends of the housing are threaded and the corresponding endcaps are also threaded so as to mate with one another,
or
(iii) the housing is in the form of a tube comprising at least one of polypropylene, polytetrafluoroethylene, or other medical grade tubing materials.

7. The system according to claim 1, wherein the adsorption chamber and/or the materials in the adsorption chamber are coated with human serum albumin and an anticoagulant added to physiological saline as a delivery solution prior to clinical use, and optionally wherein the anticoagulant is selected from the group consisting of sodium heparin and citrate dextrose solution ACD-A.

8. The system according to claim 1, wherein said adsorption chamber is effective to remove toxins other than cytokines from blood of the subject.

## Patentansprüche

1. System zum Entfernen von Zytokinen und anderen Substanzen aus dem Blut eines Subjekts (10) in einem geschlossenen Fluidkreislauf, wobei das System einen Plasmaseparator (20), eine in dem Kreislauf angeordnete Adsorptionskammer (30) und eine Vereinigungskammer (40) umfasst, wobei die Adsorptionskammer Materialien umfasst, die 50-70 Gew.-% Aktivkohle (230) und 30-50 Gew.-% nichtionisches Harz (240) umfassen, und wobei das System wirksam und dazu ausgebildet ist, die folgenden Verfahrensschritte durchzuführen:
(i) Leiten von venösem Blut (12) des Subjekts durch den Plasmaseparator, wobei der Plasmaseparator dazu ausgebildet ist, das Blut in Blutzellen (22) und Plasma (24) zu trennen;
(ii) Leiten des unmittelbar von dem Plasmaseparator erhaltenen Plasmas durch die in dem Kreislauf angeordnete Adsorptionskammer, um aufbereitetes Plasma (32) zu bilden, wobei Materialien in der Adsorptionskammer dazu ausgebildet sind, Zytokine in dem Plasma zu adsorbieren, um das aufbereitete Plasma zu bilden; und
(iii) Vereinigen des unmittelbar von der Adsorptionskammer erhaltenen aufbereiteten Plasmas mit den Blutzellen in der Vereinigungskammer, wobei die Vereinigungskammer dazu ausgebildet ist, aufbereitetes Blut (50) zu bilden, ohne dass Plasma gegen ein anderes Fluid ausgetauscht wird; und derart ausgebildet ist, dass das aufbereitete Blut aus dem Kreislauf unmittelbar in das Subjekt transfundiert werden kann, wobei dem Kreislauf kein anderes Fluid als das Blut des Subjekts zugesetzt wird, bevor die Transfusion des aufbereiteten Blutes in das Subjekt abgeschlossen werden soll; und
wobei der Plasmaseparator eine Zentrifugalapheresepumpe ist.

2. System nach Anspruch 1, wobei das nichtionische Harz mindestens ein Harzmaterial umfasst, das ausgewählt ist aus der Gruppe bestehend aus einem nichtionischen aliphatischen Esterharz, einem nichtionischen Polystyrol-Divinylbenzol-Harz, einem Agarosemedium mit hydrophober interaktiver Chromatographie und anderen nicht biologischen Adsorptionsharzen.

3. System nach Anspruch 2, wobei
(i) das nichtionische aliphatische Esterharz AMBERLITE^{®} XAD-7HP ist, oder
(ii) das nichtionische Polystyrol-Divinylbenzol-Harz AMBERCHROM^{®} GC300C ist, oder
(iii) die Aktivkohle mindestens ein Aktivkohlematerial umfasst, das ausgewählt ist aus der Gruppe bestehend aus unbeschichteter Kokosnussschalengranulatkohle, unbeschichteter organischer Granulatkohle und unbeschichtetem synthetischem Kohlenstoff.

4. System nach Anspruch 1, wobei die Adsorptionskammer aus einem Polymer aufgebaut ist, das ausgewählt ist aus der Gruppe bestehend aus Polycarbonat, Polypropylen, einem Lexan-Copolymer, Polytetrafluorethylen und anderen Polymeren in medizinischer Qualität, die für das Spritzgießen oder Blasformen geeignet sind.

5. System nach Anspruch 1, wobei die Adsorptionskammer Folgendes umfasst: ein Gehäuse (200), das ein Hohlrohr mit gegenüberliegenden offenen Enden umfasst, wobei das Gehäuse die Aktivkohle und das nichtionische Harz enthält;
poröse Membranfilter (300a,300b), die jedes der Enden (220a,220b) des Gehäuses abdecken, wobei jeder poröse Membranfilter eine Barriere bildet, um die Aktivkohle und das nichtionische Harz in dem Gehäuse zu halten und gleichzeitig den Durchtritt des Plasmas dort hindurch zu ermöglichen; und
Endkappen (400a,400b), die an jedem der Enden des Gehäuses angebracht sind, wobei jede Endkappe dazu ausgebildet ist, ihren zugehörigen porösen Membranfilter in Position zu halten und eine Abdichtung zwischen der Endkappe und dem entsprechenden Ende des Gehäuses aufrechtzuerhalten.

6. System nach Anspruch 5, wobei
(i) jede Endkappe eine Nut aufweist, die in ihren gesamten Innenumfang eingeformt ist, wobei die Nut dazu ausgebildet ist, das Zusammenfügen jeder Endkappe mit dem entsprechenden Ende des Gehäuses zu erleichtern, optional
wobei die Nut dazu ausgebildet ist, eine Menge Klebstoff aufzunehmen, und der Klebstoff in der Nut so aufgebracht ist, dass er das Anhaften des porösen Membranfilters an der Endkappe unterstützt, und ferner optional
wobei eine weitere Menge Klebstoff zwischen jeder Endkappe und dem zugehörigen porösen Membranfilter aufgebracht ist, um eine weitere Haftung zwischen der Endkappe und dem entsprechenden Ende des Gehäuses bereitzustellen, oder
(ii) die Enden des Gehäuses mit einem Gewinde versehen sind und die entsprechenden Endkappen ebenfalls mit einem Gewinde versehen sind, sodass sie ineinandergreifen,
oder
(iii) das Gehäuse in Form eines Rohrs ausgebildet ist, das mindestens eines von Polypropylen, Polytetrafluorethylen oder anderen Schlauchmaterialien in medizinischer Qualität umfasst.

7. System nach Anspruch 1, wobei die Adsorptionskammer und/oder die Materialien in der Adsorptionskammer vor der klinischen Verwendung mit Humanserumalbumin und einem Antikoagulans beschichtet sind, die einer physiologischen Kochsalzlösung als Applikationslösung zugesetzt werden, und optional wobei das Antikoagulans ausgewählt ist aus der Gruppe bestehend aus Natriumheparin und Citrat-Dextrose-Lösung ACD-A.

8. System nach Anspruch 1, wobei die Adsorptionskammer wirksam ist, um andere Toxine als Zytokine aus dem Blut des Subjekts zu entfernen.

## Revendications

1. Système d'élimination de cytokines et d'autres substances du sang d'un sujet (10) dans un circuit fluidique fermé, ledit système comprenant un séparateur de plasma (20), une chambre d'adsorption (30) située dans le circuit et une chambre de combinaison (40), dans lequel la chambre d'adsorption comprend des matériaux comprenant, en poids, 50 à 70 % de charbon actif (230) et 30 à 50 % de résine non ionique (240), et dans lequel le système est efficace et configuré pour effectuer les étapes de procédé suivantes :
(i) faire passer du sang veineux (12) provenant du sujet à travers le séparateur de plasma, dans lequel le séparateur de plasma est configuré pour séparer le sang en cellules sanguines (22) et en plasma (24) ;
(ii) faire passer le plasma directement reçu du séparateur de plasma à travers la chambre d'adsorption située dans le circuit afin de former du plasma traité (32), dans lequel des matériaux contenus dans la chambre d'adsorption sont configurés pour adsorber des cytokines présentes dans le plasma afin de former le plasma traité ; et
(iii) combiner le plasma traité, reçu directement de la chambre d'adsorption, avec les cellules sanguines dans la chambre de combinaison, dans lequel la chambre de combinaison est configurée pour former du sang traité (50), sans échanger la moindre partie du plasma contre un autre fluide ; et configurée de sorte que le sang traité provenant du circuit puisse être transfusé directement au sujet, dans lequel aucun fluide autre que le sang du sujet n'est ajouté au circuit avant que la transfusion du sang traité au sujet ne soit achevée ; et
dans lequel le séparateur de plasma est une pompe d'aphérèse centrifuge.

2. Système selon la revendication 1, dans lequel la résine non ionique comprend au moins un matériau de résine choisi dans le groupe constitué d'une résine d'ester aliphatique non ionique, d'une résine de polystyrène-divinylbenzène non ionique, d'un milieu d'agarose pour chromatographie d'interaction hydrophobe, et d'autres résines adsorbantes non biologiques.

3. Système selon la revendication 2, dans lequel
(i) la résine d'ester aliphatique non ionique est l'AMBERLITE^{®} XAD-7HP, ou
(ii) la résine de polystyrène-divinylbenzène non ionique est l'AMBERCHROM^{®} GC300C, ou
(iii) le charbon actif comprend au moins un matériau de charbon actif choisi dans le groupe constitué de charbon sous forme de granulés de coque de noix de coco non enrobé, de charbon sous forme de granulés organiques non enrobé, et de charbon synthétique non enrobé.

4. Système selon la revendication 1, dans lequel la chambre d'adsorption est construite à partir d'un polymère choisi dans le groupe constitué du polycarbonate, du polypropylène, d'un copolymère de Lexan, du polytétrafluoroéthylène, et d'autres polymères de qualité médicale adaptés au moulage par injection ou par soufflage.

5. Système selon la revendication 1, dans lequel la chambre d'adsorption comprend : un boîtier (200) comprenant un tube creux doté d'extrémités ouvertes opposées, ledit boîtier contenant le charbon actif et la résine non ionique ;
des filtres à membrane poreuse (300a, 300b) recouvrant chacune des extrémités (220a, 220b) du boîtier, chaque filtre à membrane poreuse créant une barrière pour maintenir le charbon actif et la résine non ionique à l'intérieur du boîtier tout en permettant le passage du plasma à travers celui-ci ; et
des embouts (400a, 400b) emboîtés sur chacune des extrémités du boîtier, dans lequel chaque embout est configuré pour maintenir en place son filtre à membrane poreuse correspondant et pour maintenir une étanchéité entre l'embout et l'extrémité correspondante du boîtier.

6. Système selon la revendication 5, dans lequel
(i) chaque embout comporte une rainure moulée sur l'ensemble de sa circonférence intérieure, ladite rainure étant configurée pour faciliter l'assemblage de chaque embout avec l'extrémité correspondante du boîtier, éventuellement
dans lequel ladite rainure est configurée pour recevoir une quantité d'adhésif, et ledit adhésif étant déposé dans la rainure de manière à aider à l'adhérence du filtre à membrane poreuse sur l'embout, et en outre éventuellement
dans lequel une autre quantité d'adhésif est déposée entre chaque embout et son filtre à membrane poreuse correspondant afin d'assurer une adhérence supplémentaire entre l'embout et l'extrémité correspondante du boîtier, ou
(ii) les extrémités du boîtier sont filetées et les embouts correspondants sont également filetés de manière à s'assembler les uns avec les autres,
ou
(iii) le boîtier se présente sous la forme d'un tube comprenant au moins l'un parmi le polypropylène, le polytétrafluoroéthylène ou d'autres matériaux pour tubulure de qualité médicale.

7. Système selon la revendication 1, dans lequel la chambre d'adsorption et/ou les matériaux contenus dans la chambre d'adsorption sont revêtus d'albumine sérique humaine et d'un anticoagulant ajouté à du sérum physiologique en tant que solution d'administration avant toute utilisation clinique, et éventuellement dans lequel l'anticoagulant est choisi dans le groupe constitué de l'héparine sodique et de la solution de citrate-dextrose ACD-A.

8. Système selon la revendication 1, dans lequel ladite chambre d'adsorption est efficace pour éliminer du sang du sujet des toxines autres que les cytokines.
